# EUROPEAN PATENT APPLICATION

(11) **EP 4 733 950 A1**
(43) Date of publication of application: **29.04.2026**
(21) Application number: 25205043.0
(22) Date of filing: 26.09.2025
(51) Int. Cl.: G06F 16/11

(54) **HUMAN FACTOR DATA STORAGE METHOD AND ANALYSIS METHOD, ELECTRONIC DEVICE AND STORAGE MEDIUM**

(30) Priority: 28.10.2024 CN 202411515397
(71) Applicant: Kingfar International Inc., Beijing 100193 (CN)
(72) Inventor: ZHAO, Qichao, Beijing, 100193 (CN); YANG, Ran, Beijing, 100193 (CN)
(74) Representative: Lorenz Seidler Gossel Part. mbB

(57) **Abstract**

The present disclosure provides a human factor data storage method and analysis method, an electronic device and a storage medium. The storage method includes: acquiring a data packet from a sensor, the data packet containing human factor data; and storing the human factor data in a human factor data file in a predetermined data format, wherein the predetermined data format has a file header and a data body, the file header includes a frame header, a length value and a check value, the human factor data from the sensor is stored in the data body, and the check value in the file header is obtained based on a predetermined check algorithm. The human factor data storage method according to the present disclosure can keep data integrity and has a high data storage efficiency, and correspondingly, the analysis method has a high analysis efficiency. Data safety can be further improved.

## Description

### CROSS-REFERENCE TO RELATED APPLICATION

This application claims priority to Chinese Patent Application No. CN202411515397 .8 filed on October 28, 2024, which is hereby incorporated by reference in its entirety.

### TECHNICAL FIELD

The present disclosure relates to the field of human factors engineering and human ergonomics technologies, and in particular, to a human factor data storage method and analysis method, an electronic device and a storage medium.

### BACKGROUND

Human factors engineering and human ergonomics mainly research relationships among humans, machines and environments, comprehensively utilize physiology, psychology, hygiene, anthropometry, or the like, to research interactions between the humans and other elements of a system, and get more and more attention. Human factors engineering and human ergonomics research the reasonable relationships among the humans, the machines and the environments according to factors of human psychology, physiology, body structures, and the like, so as to ensure that people work safely, healthily and comfortably and obtain satisfactory working effects, and therefore play increasingly significant roles in improving physical and mental health, working efficiencies, safety and comfort of the people. Data of human bodies, the machines, human-machine interactions, the environments, and the like, collected during analysis of human behaviors, cognition and operations in the human factors engineering can be called human factor data. Instruments for collecting the human factor data may include various types of sensing devices or other measuring devices to collect various kinds of physiological data, psychological data, behavioral data, and the like. The human factor data widely relates to various application fields, such as health, medical treatment and intelligent driving, and storage, processing and complex queries of mass human factor data, data safety, and the like, become more and more important. Therefore, how to design a novel data storage method, especially a data storage method suitable for the human factor data and a corresponding analysis method, to improve data storage and access efficiencies becomes a problem to be solved urgently.

### SUMMARY

**In** view of this, embodiments of the present disclosure provide a human factor data storage method, a human factor data analysis method and an electronic device, so as to obviate or improve one or more disadvantages in the prior art.

**In** an aspect, the present disclosure provides a human factor data storage method, including the following steps:
acquiring a data packet from a sensor, the data packet containing human factor data; and
storing the human factor data in a human factor data file in a predetermined data format, wherein the predetermined data format has a file header and a data body, the file header includes a frame header, a length value and a check value, the human factor data from the sensor is stored in the data body, and the check value in the file header is obtained based on a predetermined check algorithm.

**In** some embodiments of the present disclosure, the file header further includes a file header payload, and the file header payload includes sensor description fields for representing sensor features; the length value in the file header is the length of the file header payload.

**In** some embodiments of the present disclosure, the method further includes: before storing the human factor data from the sensor for the first time, receiving a message from the sensor containing the sensor description fields, and generating the file header of the human factor data file based on the received message.

**In** some embodiments of the present disclosure, the generating the file header of the human factor data file based on the received message includes: taking a message body from the sensor containing the sensor description fields as the file header, the message body including the frame header, the length value, the check value and the payload, and the payload including the plurality of sensor description fields; or obtaining the plurality of sensor description fields based on the message from the sensor containing the sensor description fields, filling the frame header of the file header, and filling the length value, the check value and the payload in the file header based on the plurality of sensor description fields to obtain the file header.

In some embodiments of the present disclosure, the data packet includes a packet header part and a payload part, the packet header part includes a frame header, a payload length value and a check value, and the payload part of the data packet includes the human factor data.

In some embodiments of the present disclosure, the storing the human factor data in a human factor data file in a predetermined data format includes: storing the data packet from the sensor as a whole in the data body of the human factor data file, the data body containing one or more data packets.

In some embodiments of the present disclosure, the storing the human factor data in a human factor data file in a predetermined data format includes: checking the obtained data packet based on the predetermined data packet check algorithm and the check value in the data packet, and obtaining the human factor data in the data packet after successful checking; and storing the obtained human factor data in the data body of the human factor data file.

In some embodiments of the present disclosure, the sensor description fields include some or all of the following fields: sensor ID, sensor type, sensor name suffix and data packet reporting frequency.

In some embodiments of the present disclosure, the data packet is a data packet from a human factor data collection sensor containing human factor data; the human factor data file performs storage based on a hexadecimal format.

In some embodiments of the present disclosure, an operation start instruction is sent to the sensor to instruct the sensor to send the data packet containing the human factor data before the acquiring of the data packet from the sensor.

In some embodiments of the present disclosure, the message from the sensor containing the sensor description fields is received based on the following interaction process: sending an initialization message to the sensor and receiving an initialization message from the sensor, the initialization message including a frame header region, a payload length region, a check region and a message body part, and the message body part including a message type and sensor initialization information including a the number of connected sensors, type of the sensor and group information; and based on the received initialization message from the sensor, sending a scan command message to the sensor, the scan command message including a frame header region, a payload length region, a check region and a message body part, the message body part including a message type and the plurality of sensor description fields, and the plurality of sensor description fields including some or all of the following fields: sensor ID, sensor type, sensor name suffix and data packet reporting frequency.

In another aspect, the present disclosure provides a human factor data analysis method, the human factor data being stored in a human factor data file in a predetermined format, the predetermined data format having a file header and a data body, the file header including a frame header, a length value and a check value, the data body including the human factor data, and the method including: a file header analysis step: checking the human factor data file header based on a predetermined check algorithm and the check value in the file header, and reading other information in the file header after the checking is successful; and a data body analysis step: after the successful file header checking, reading the human factor data in the data body.

In some embodiments of the present disclosure, the data body contains at least one data packet containing the human factor data, the data packet containing the human factor data includes a packet header and a payload, the packet header includes a frame header, a payload length value and a check value, a payload of the data packet includes the human factor data, and the human factor data analysis step includes: checking the data packet in the data body based on a predetermined data packet check algorithm and the check value in the data packet, and reading the human factor data in the data packet after successful checking.

In some embodiments of the present disclosure, the file header further includes a file header payload, and the file header payload includes sensor description fields; and the method further includes: displaying the read information in the file header and the human factor data in the data body on a display apparatus.

In another aspect, the present disclosure provides an electronic device, including a processor, a memory and computer instructions stored on the memory, the processor being configured to execute the computer instructions, and the device implementing the steps of the method as described above when the computer instructions are executed.

In another aspect, the present disclosure further provides a computer-readable storage medium having computer instructions stored therein, the computer instructions, when executed by a processor, implementing the steps of the method as described above.

The human factor data storage method and the corresponding electronic device according to the present disclosure ensure a high data storage efficiency and high data safety and can adapt to diversified human factor data. Correspondingly, the human factor data analysis method according to the present disclosure has a high analysis efficiency and high safety and facilitates access.

Additional advantages, objects and features of the present disclosure will be set forth in part in the description which follows and in part will become apparent to those having ordinary skill in the art upon examination of the following or may be learned from practice of the present disclosure. The objects and other advantages of the present disclosure will be achieved and attained by the structure particularly pointed out in the specification and drawings.

It will be appreciated by those skilled in the art that the objects and advantages that can be achieved with the present disclosure are not limited to what has been particularly described hereinabove, and that the above and other objects that can be achieved with the present disclosure will be more clearly understood from the following detailed description.

### BRIEF DESCRIPTION OF THE DRAWINGS

The accompanying drawings described herein are used to facilitate further understanding of the present disclosure, constitute a part of the present application, and do not limit the present disclosure.
FIG. 1 is a schematic flow diagram of a human factor data storage method according to an embodiment of the present disclosure.
FIG. 2 shows a structural example of a data packet in an embodiment of the present disclosure.
FIG. 3 is a schematic diagram of a human factor data storage process in an embodiment of the present disclosure.
FIG. 4 is a schematic diagram of the human factor data storage process in another embodiment of the present disclosure.
FIG. 5 is a schematic diagram of the human factor data storage process in still another embodiment of the present disclosure.
FIG. 6A and FIG. 6B are structural examples of data bodys in human factor data files in the embodiments of the present disclosure.
FIG. 7 is a schematic flow diagram of a human factor data analysis method according to an embodiment of the present disclosure.
FIG. 8 is a schematic diagram of a human factor data analysis process in an embodiment of the present disclosure.
FIG. 9 is a schematic diagram of the human factor data analysis process in another embodiment of the present disclosure.
FIG. 10 is a schematic structural diagram of a CmdInfo message body transmitted in an embodiment of the present disclosure.
FIG. 11 is a schematic structural diagram of a CmdScan message body transmitted in an embodiment of the present disclosure.
FIG. 12 is a schematic structural diagram of a CmdStart message body transmitted in an embodiment of the present disclosure.

### DETAILED DESCRIPTION

In order to make the objects, technical solutions and advantages of the present disclosure more apparent, the present disclosure is described in further detail below with reference to the embodiments and the accompanying drawings. Herein, the exemplary embodiments of the present disclosure and the descriptions thereof are used to explain the present disclosure, but not to limit the present disclosure.

It should also be noted herein that, in order to avoid obscuring the present disclosure with unnecessary details, only the structures and/or processing steps closely related to the solution according to the present disclosure are shown in the drawings, and other details not so related to the present disclosure are omitted.

It should be emphasized that the term "includes/comprises", when used herein, is taken to specify the presence of features, elements, steps or components, but does not preclude the presence or addition of one or more other features, elements, steps or components.

Aiming at the problems of an efficiency, safety, and the like, of an existing data storage format in human factor data storage and subsequent analysis, the present disclosure provides a novel human factor data storage method and a corresponding human factor data analysis method, wherein the human factor data includes human body-related data, machine-related data, human-machine interaction-related data, and environment-related data. The human body-related data includes, but is not limited to, one or a combination of: skin conductance and skin temperature data, pulse data, blood pressure data, blood oxygen data, electrocardiogram data, electromyography data, muscle oxygen data, respiratory data, biomechanical data, near-infrared brain imaging data, electroencephalogram data, transcranial stimulation data, heart rate variability data, heart rate data, image/video data of human, sound data of human, eye movement data, and gesture or action data. The machine-related data includes, but is not limited to, one or a combination of: machine running data, fault alarm data, machine manipulation data, machine model data, machine communication data and machine positioning data. The human-machine interaction-related data includes, but is not limited to, one or a combination of: human-machine voice interaction data, human-machine text interaction data, human-machine touch interaction data, human-machine gesture or action interaction data, human-machine electroencephalogram interaction data, human-machine eye movement interaction data and human-machine expression interaction data. The environment-related data includes, but is not limited to, one or a combination of: position data, humidity data, temperature data, chromaticity data, brightness data, weather data, road condition data, traffic data, stimulus signal data, and event or signal marking data. Such data may be acquired by sensing devices or other types of measuring devices that support collection of corresponding types of data. For ease of description, the human factor data collection and acquisition device is referred to as a "sensor" in the present disclosure.

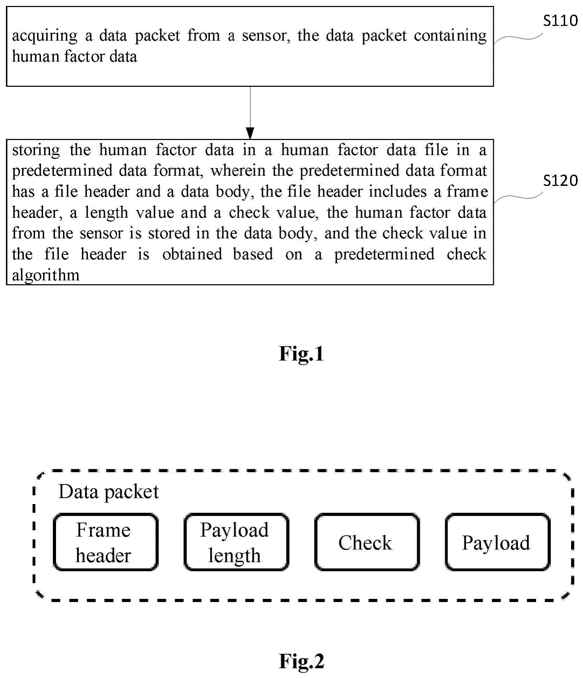

FIG. 1 shows a schematic flow diagram of a human factor data storage method according to an embodiment of the present disclosure, the method may be executed by a storage device (such as a computer, a mobile phone, other dedicated handheld terminals, or the like) that interacts with the sensor and receives sensor data, the following description is given with the mobile phone as the storage device as an example, but the storage device in the present disclosure is not limited to the mobile phone, and may be other terminals that interact with the sensor and have a data storage function. The storage device may be communicated with the sensor directly or communicated with a plurality of sensors via a hub, or the like. After receiving a data packet from the sensor, a storage program of the mobile phone can execute the human factor data storage method according to the present disclosure. As shown in FIG. 1, the human factor data storage method includes the following steps:
step S110: acquiring the data packet from the sensor, the data packet containing human factor data.

In the embodiment of the present disclosure, sensed data of various types of sensors, that is, sensed data of various types of sensors capable of obtaining human factor data, can be acquired, and the sensed data of the sensors can be contained in one data packet or divided into a plurality of data packets which are transmitted to the mobile phone and stored by the mobile phone. Examples of the sensor may include a pressure sensor, an acceleration sensor, a gyroscope sensor, an electrocardiogram sensor, an electromyography sensor, an angular velocity sensor, a pulse sensor, and the like, but the present disclosure is not limited thereto. One sensor can also collect different types of sensed signals through multiple channels to obtain multiple types of sensed data, for example, can simultaneously obtain posture data, temperature data, and the like.

Step S120: storing the human factor data in a human factor data file in a predetermined data format, wherein the predetermined data format has a file header and a data body, the file header includes a frame header, a length value and a check value, and the human factor data from the sensor is stored in the data body.

In some other embodiments of the present disclosure, the frame header in the file header is a value that identifies the sensor type or the sensed data type, such as a sensor ID or a sensed data type, but the present disclosure is not limited thereto. The check value in the file header is obtained based on a predetermined check algorithm, and the predetermined check algorithm may be an XOR check algorithm or other existing check algorithms. For example, when the check algorithm is the XOR check algorithm, check information for performing a check operation may be predetermined. Since the check algorithm is a mature technology, it is not repeated herein. The length value in the file header may represent a length value of a payload in the file header, or may be a length value of a part or the whole of the data body in the human factor data file.

In the case where the length value in the file header represents the length value of the payload in the file header, the file header includes, in addition to the frame header, the length value and the check value, the file header payload, the file header payload may include sensor description fields for representing sensor features, the sensor features include, but are not limited to, features of the sensor itself and/or features of the sensed data of the sensor, the features of the sensor itself may include, for example, some or all of the following features: a sensor identification feature (e.g., sensor ID), a sensor type feature, a sensor name feature (e.g., sensor name suffix), a group to which the sensor is belonged, and the like, and the features of the sensed data of the sensor may include, for example, some or all of the following features: the sensed data type, a data packet reporting frequency, and the like. As an example, the sensor description fields include some or all of the following fields: the sensor ID, the sensor type, the sensor name suffix and the data packet reporting frequency, but the present disclosure is not limited thereto. In the case where the length value in the file header represents the length value of the file header payload, the file header payload can be quickly read during data analysis based on the length value.

In some other embodiments of the present disclosure, in the case where the file header includes the file header payload, the length value of the file header payload may be a predetermined fixed length, and in this case, the length value in the file header may be an entire length of the data body in the file.

In an alternative embodiment of the present disclosure, the file header may not contain the payload, the sensor description field for representing the sensor feature may be stored as the payload in the data body of the file when the human factor data is stored for the first time, and in this case, the length value in the file header preferably represents only a length of the sensor description field in the data body, though it may also represent the entire length of the data body in the file.

In some embodiments of the present disclosure, the file header may be generated when the data packet from the sensor containing the human factor data is received for the first time and the data is to be stored, the file header may also be generated in advance before the data packet containing the human factor data is received, the file header may be generated and stored only once, and is not required to be changed in a subsequent storage process, or only a length value field in the file header is updated when the length value field in the file header should be updated. The data body of the human factor data file usually performs storage a plurality of times, and every time the data packet from the corresponding sensor containing the human factor data is received, the whole data packet or the human factor data in the data packet is stored into the data body in sequence.

In some embodiments of the present disclosure, every time the data packet from the sensor is received, the storage device, such as the mobile phone, may store the received complete data packet in the data body of the human factor data file, or extract (read) the human factor data in the data packet, and then store the read human factor data in the data body of the human factor data file.

The present disclosure ensures a high data storage efficiency and data safety by storing the human factor data into the human factor data file in the predetermined data format. The predetermined data format has the file header and the data body, and since the file header includes the check value, the data safety can be ensured. The human factor data from the same sensor can be stored in the data body of a same human factor data file, and the data storage efficiency is high.

In addition, in a preferred embodiment of the present disclosure, the human factor data file performs storage in a hexadecimal format, and compared with a binary data storage format, the data storage efficiency can be further improved.

In some embodiments of the present disclosure, the file header of the human factor data file may be generated based on sensor description fields stored by the storage device in advance before the data packet from the sensor containing the human factor data is stored for the first time, or may be generated based on a message from the sensor containing sensor description fields before the data packet from the sensor containing the human factor data is stored for the first time. If the file header is generated based on the message from the sensor containing sensor description fields, the method further includes the following step:

Before storing the data packet from the sensor containing the human factor data, receiving the message from the sensor containing sensor description fields, and generating the file header of the human factor data file based on the received message. The sensor description fields used to generate the file header are preferably a plurality of fields representing different sensor description information, and may be obtained by receiving one or more messages from the sensor containing the sensor description fields.

The data packets from the sensors acquired in the present disclosure can be data packets transmitted based on different data transmission protocols, and the data packets can be allowed to have different structures based on different data transmission protocols.

As an example, the data packet from the sensor is a data packet transmitted based on a data transmission protocol between the sensor and an host computer, or the like. As shown in FIG. 2, the data packet may include: a packet header part and a payload (data content which can be called "load" for short) part, the packet header part is a part that identifies a beginning of the data packet and includes: a frame header region, a payload length region and a check region, the payload length represents the length of the payload, the check value in the check region is a value calculated with a check algorithm to prevent a data error, and a receiver can check whether an error occurs in a data transmission process based on a pre-negotiated check algorithm, so as to respond in time when the error is found. In an optional application example, the payload length in a packet header may be set based on the length occupied by actual human factor data, so that a numerical value of the payload length is dynamically adjustable, and correspondingly, since the number of bytes occupied by the payload length region corresponds to the numerical value of the load length, the number of bytes occupied by the data content in the load part is also dynamically adjustable, thereby satisfying requirements of the transmission of the human factor data with different load lengths. A device receiving the human factor data can store and/or analyze the human factor data by adopting the method according to the present disclosure.

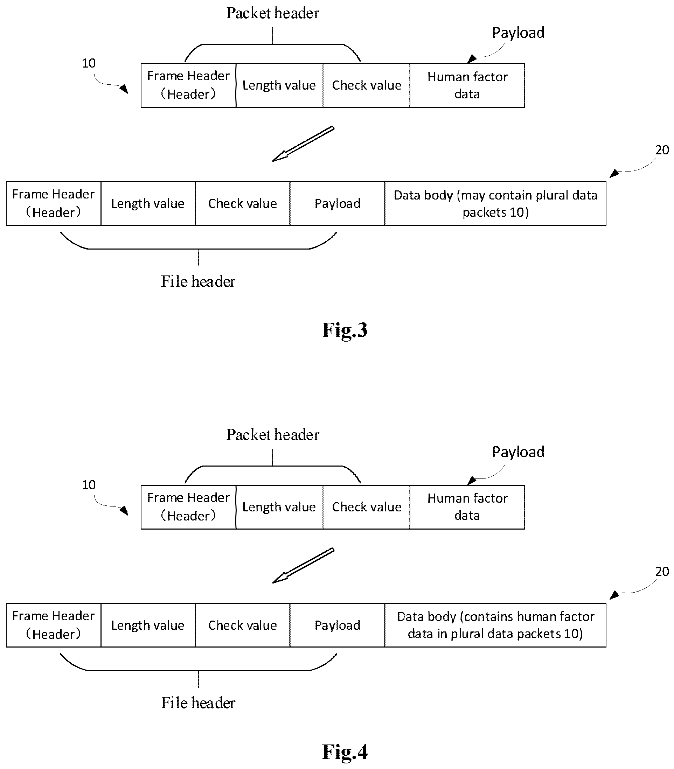

FIG. 3 to FIG. 4 are schematic diagrams of a data storage process in different embodiments of the present disclosure. Referring to FIG. 3 to FIG. 4, the data storage process in the present embodiment is as follows:
step S11: receiving a data packet 10 from a sensor containing human factor data.

The data packet 10 includes a packet header and a payload, the packet header may include a frame header, a length value and a check value, the payload includes the human factor data, the length value in the packet header represents a length of the payload, and the payload contains the human factor data of one or more channels.

Step S12: generating a file header of a human factor data file in a predetermined data format.

In an initial condition, the file header and a data body of the human factor data file in the predetermined data format are both empty. The file header of the human factor data file in the predetermined data format includes a frame header region, a length region and a check region, and preferably further includes a payload region, the payload region includes a sensor description field used to be filled with a sensor feature, typically a plurality of sensor description fields, such as a sensor ID, a sensor type, a sensor name suffix, and a data packet reporting frequency. In this step, before or after the data packet containing the human factor data is received and before the human factor data is stored, the data packet carrying sensor description information (with the sensor description field as the payload) and received from the sensor may be directly used as the file header, or the initially empty file header may be filled based on information acquired in advance.

More specifically, if the sensor description information is stored in advance in a storage device, such as a mobile phone, or is provided to the mobile phone by the sensor through at least one interaction between the mobile phone and the sensor, in this step, values of the regions of the file header may be generated through the obtained sensor description information to fill the file header, so as to obtain the file header of the human factor data file in the predetermined data format. The frame header of the file header may have a fixed value, for example, the sensor ID may be used as the frame header, and certainly, other information may be used as the frame header, and the length of the frame header in the file header may be a fixed value (e.g., 1 byte or other values) or may be preset. The check value may be obtained with a predetermined check algorithm (e.g., an XOR algorithm) based on the sensor description fields, the payloads may be the sensor description fields, and the length value may be a payload length, i.e., the length value of the sensor description fields.

If the sensor description information is transmitted to the mobile phone by the sensor through one data packet at one time, the mobile phone can directly use the data packet from the sensor carrying the sensor description information as the file header of the human factor data file. To perform this step, the mobile phone may request the sensor description information from the sensor and receive the data packet from the sensor carrying the sensor description information (with the sensor description field as the payload) before the step S11. Since the data packet includes the frame header, the length value, the check value and the payload, the data packet with the sensor description field as the payload can be directly used as the file header of the human factor data file in this step, and at this point, the frame header, the length value, the check value and the payload in the file header are completely the same as those in the data packet with the sensor description information.

The file header can be generated only once, and when the human factor data is stored into the data body subsequently, the file header does not need to be generated repeatedly.

In the embodiment of the present disclosure, in addition to the fields of the frame header, the length value, the check value and the payload (optional), other fields may be flexibly included in the file header based on scenarios, such as a sensor identification field and a measurement item field, and the present disclosure is not limited thereto.

Step S13: storing the human factor data contained in the received data packet 10 in the data body.

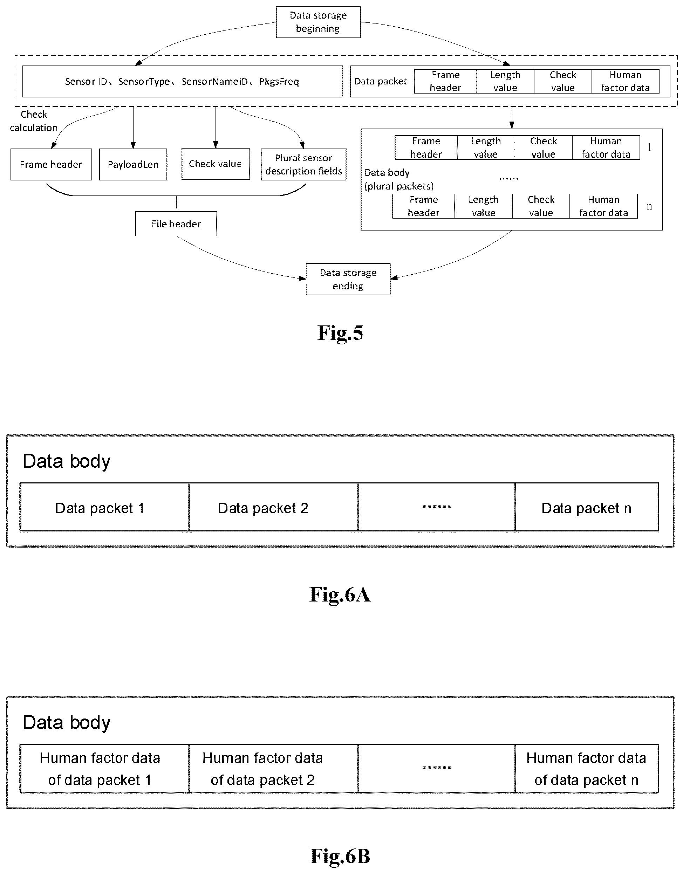

In this step, the received data packet 10 containing the human factor data may be directly stored in the data body without checking the data packet, and at this point, as shown in FIG. 3 and FIG. 6A, one or more data packets are contained in the data body. Alternatively, it is also possible to extract the human factor data from the data packet containing the human factor data and then store the extracted human factor data in the data body, and as shown in FIG. 4 and FIG. 6B, the data body only contains the human factor data in the data packet rather than the entire data packet. Under the condition of extracting and storing the human factor data, the data packet needs to be checked with the predetermined check algorithm first, and after the checking is successful, the human factor data is read based on the length value in the packet header and is stored in the data body. Whether the data packet is sent wrongly can be identified through checking, thus guaranteeing accuracy of data transmission. If the checking fails, it indicates that the data packet was wrongly sent and can be discarded.

FIG. 5 shows a more specific example of the data storage process of the present disclosure. As shown in FIG. 5, before the mobile phone receives the data packet containing the human factor data from the sensor and stores the human factor data, whether the file header is generated is confirmed, and if the file header is generated, the data packet containing the human factor data is directly stored in the data body; if the file header is not generated, that is, the data packet is the first data packet to be stored, the file header is first generated by the following operations:
1) using the sensor ID as the frame header;
2) performing check calculation based on the predetermined check algorithm to obtain a check value as the check value in the file header, for example, performing check calculation using the sensor ID (Sensor ID), the sensor type (SensorType), the sensor name suffix (SensorNameID), and the data packet reporting frequency (PkgsFreq) to generate the check value in the file header;
3) using currently known sensor description information, such as the sensor ID (Sensor ID), the sensor type (SensorType), the sensor name suffix (SensorNameID), and the data packet reporting frequency (PkgsFreq), as the payloads in the file header, the length value being the length of these payloads.

In the above step of forming the file header, each of the frame header, the length value and the check value may only occupy 1 byte, and as an example, hexadecimal 0xFC, 0xFB and 0xFA may represent different sensor system types, 0xFC represents a Bluetooth-based wireless sensor system, 0xFB represents an electroencephalogram physiological collection system, 0xFA represents a functional near infrared system, and if 1 byte is used to represent the sensor system type as the frame header, the frame header region may be filled with C, B or A only to represent the corresponding 0xFC, 0xFB or 0xFA. The lengths of the fields in the file header may be set to other lengths as appropriate based on actual application scenarios. Contents in the file header and/or the data body can be encrypted by adopting an obfuscated encryption manner or other encryption manners, so as to further improve the data security.

After the file header is generated, the data packet containing the human factor data is stored in the data body, thereby finishing storage of the current data packet. Under the condition that the data packets are stored in the data body, when the human factor data file is analyzed subsequently by the host computer, the data packets in the data head and the data body need to be checked one by one, the data packet with wrong checking is discarded, and data in the data packet with correct checking is analyzed and displayed.

As a variant embodiment, the sensor description information in the file header as the payload may also be stored in the data body, and only needs to be stored once at first storage, and the data packet containing the human factor data is stored behind the sensor description information. As a variant embodiment, the data body may also store other necessary data based on a specific scenario in addition to the sensor description information and the human factor data.

As a variant embodiment, the length value in the file header may also be the entire length of the content in the data body.

As a variant embodiment, the mobile phone may check the data packet containing the human factor data, extract the human factor data in the data packet after the checking is successful, and only store the human factor data in the data body. In this case, when the host computer subsequently analyzes the human factor data file, the content of the data body does not need to be checked, and only the data header needs to be checked.

The data body in the human factor data file in the present disclosure can contain the human factor data of one data packet, or contain the human factor data of a plurality of data packets. When the human factor data in the plural data packets is stored in the same human factor data file, the file header can perform storage only once, the data body can perform storage many times, and the data in the data packets is stored in sequence. If the length in the file header represents the length of the data body, the length value in the file header may be updated every time the length of the data body changes.

The hexadecimal format is adopted for data storage of the human factor data file in the present disclosure, each hexadecimal number represents 4 bits, and compared with a binary data storage format, hexadecimal data representation is more compact, and the storage efficiency is higher. Furthermore, some or all of the frame header, the length value, the check value, or the like, in the file header may be set as encrypted fields, and the encryption includes obfuscated encryption. In the case where the file header further includes other fields, the other fields may be set as encrypted fields as well. Thus, the data safety can be guaranteed.

In summary, in the data storage method according to the present disclosure, data format conversion is avoided, the human factor data in the data packet can be efficiently stored in the human factor data file in the predetermined data format, and the data safety is high; in addition, the data format is simple and a query is easy.

Before storing the human factor data, the host computer can interact with the sensor multiple times to obtain the sensor information. For example, message types (CmdType) include CmdInfo (initialization information), CmdScan (scan command), CmdScanAck (scan command acknowledgment), CmdEventInfo (marking information), CmdStart (data start), and the like.

### (1) CmdInfo (initialization information) message

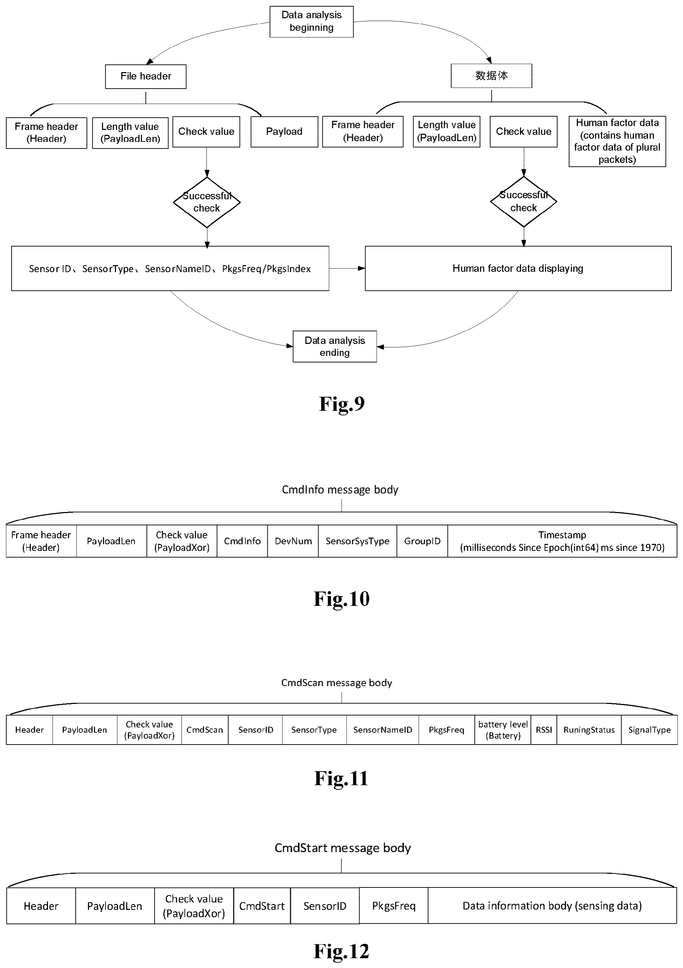

An initial initiator of the CmdInfo message can be the mobile phone, a receiver may be the sensor, and the CmdInfo message is used to obtain an initialization information field of the sensor. A structure of a CmdInfo message body is shown in FIG. 10, and includes the following fields: frame header (Header), payload length (PayloadLen), check value (PayloadXor) and payloads (Payload), and the payloads include: message type (CmdType, specifically CmdInfo), test device number (DevNum), sensor system type (SensorSysType), group number (GroupID), and timestamp (milliseconds Since Epoch(int64) ms since 1970). The first byte (Byte_0) in the CmdInfo message is the frame header, the second byte (Byte_1) is the payload length, and if the payload length is C, the length is 12, the third byte (Byte_2) is the check value, and PayloadXor indicates the check value obtained by XOR checking on the the payloads. The payload starts with the fourth byte (Byte_3). CmdInfo is a specific message type (CmdType) for confirming whether a test device (sensor) exists, a value of the message type CmdInfo is predefined, and a receiving end of the message performs matching of the message type based on the value. DevNum which is 0 indicates that the sensor is not connected through a hub, and DevNum which is another value represents the number of the sensors connected through the hub. SensorSysType may represent a plurality of meanings, such as a bandwidth, presence or absence of a battery, wired transmission or wireless transmission, and different meanings may be represented by preset values. GroupID represents groups in which a transmitting end and the receiving end (the sensor and the mobile phone) are located, and when in the same group, the transmitting end and the receiving end can communicate and receive and transmit messages, and when in different groups, the transmitting end and the receiving end cannot communicate with each other. The timestamp represents an operation time, which is a data operation time obtained by first using a programmer calculator to calculate a time of the sensing device and then converting the time of the sensing device into milliseconds, and the timestamp is mainly used for time alignment with the sensor. In the CmdInfo message sent by the mobile phone to the sensor, the field needing to be filled by the sensor is initially empty, and after the sensor receives the CmdInfo message and performs filling, the CmdInfo message (also called a CmdInfo acknowledgment message) is further returned to the mobile phone.

More specifically, after receiving the CmdInfo message from the mobile phone, the sensor performs group number checking, and if a group number is the same, the CmdInfo message can be returned to the mobile phone, which indicates that a further scanning operation can be performed.

### (2) CmdScan (scan command) message

An initial initiator of the CmdScan message may be the mobile phone, a receiver may be the sensor, and the CmdScan message is used to instruct the sensor to start to perform the scanning operation, and request all sensors in a specified group to report respective identification information. The sensor starts the scanning operation after receiving the scan command message, and can transmit information, such as the sensor ID, a main purpose and a measurement item of the sensor device, a sensor name suffix, a sampling rate, a battery level, and a Bluetooth signal, to the mobile phone through a scan command acknowledgment message.

A structure of a CmdScan message body is shown in FIG. 11, and includes the following fields: frame header (Header), load length (PayloadLen), check value (PayloadXor) and payloads (Payload), and the payloads include: message type (CmdType, specifically CmdScan), sensor number (Sensor ID), sensor type (SensorType), sensor name suffix (SensorNameID), data packet reporting frequency (PkgsFreq), battery level (Battery), signal strength (RSSI), running status (RunningStatus), signal type (SignalType), or the like. CmdScan is a specific message type (CmdType) for requesting all the sensors in the specified group to report respective identification information, the value of the message type CmdScan is predefined, and the receiving end of the message performs matching of the message type based on the value. The sensor number is used to identify the sensor. The sensor type (SensorType) is used to identify the main purposes and the measurement items of individual sensor device. When the sensor name suffix is 0, it indicates that the name suffix will not be displayed on the host computer.

In the CmdScan message sent by the mobile phone to the sensor, the field needing to be filled by the sensor is initially empty, and after the sensor receives the CmdScan message and performs filling, the CmdScan message is further returned to the mobile phone.

### (3) CmdScanAck (scan command acknowledgment) message

The initial initiator of the CmdScanAck message can be the mobile phone, and the receiver can be the host computer. After the mobile phone confirms that the scanning operations of all the sensors in the group are received, the number of the sensors reporting their identification information is feedback to the host computer through the CmdScanAck message.

A CmdScanAck message body may include the following fields: frame header, payload length, check value and payloads, and the payloads can include: message type (CmdType, specifically CmdScanAck), group number (GroupID), number of test devices (DevNum), or the like.

### (4) Marking information (CmdEventInfo) message

An initial initiator of the CmdEventInfo message can be the mobile phone, and a receiver can be the sensor.

The CmdEventInfo message may include the following fields: frame header, payload length, check value and payloads, the payloads can include: message type (CmdType, specifically CmdEventInfo), event type (EventType), or the like, and the CmdEventInfo message is used for performing behavior (event) marking.

Marking information is not essential and may be set according to a specific scenario, and the marking information will not be transmitted when not required. Scenarios where behavior marking may be performed include, for example, a running start, a running end, or the like. The marking information indicates that a marking operation of a certain event may be performed on the mobile phone, and marking information recording is instructed to be started by clicking a marking button.

After receiving the marking information message, the sensor starts a corresponding data measurement operation or status recording operation, and returns corresponding measurement data to the mobile phone in the form of a data packet containing human factor data after a marking event is finished.

### (5) Start command (CmdStart) message

An initial initiator of the start command (CmdStart) message may be the mobile phone, a receiver may be the sensor, and the message is used to send a start command to the sensor and instruct the sensor to start to perform an operation or report collected data. The sensor confirms that the start command is received, starts to execute the operation, and transmits the sensor number and collected data information to the mobile phone by returning the CmdStart message to the mobile phone.

A structure of the CmdStart message is shown in FIG. 12 and includes the following fields: frame header, load length, check value and payloads, and the payloads include: message type (CmdType, specifically CmdStart), sensor number (Sensor ID), data packet index (PkgsIndex), data information body (human factor data), and the like. CmdStart is used for requesting the sensor to collect and report data. The data packet index is used for identifying the data packet, and whether a missing packet or an error packet exists in data packet transmission can be checked based on the data packet index.

### (6) Stop command (CmdStop) message

An initial initiator of the stop command (CmdStop) message may be the mobile phone, a receiver may be the sensor, and the message is used to send a stop command to the sensor and instruct the sensor to stop current operations or data collection. The sensor confirms that the stop command is received, then stops the corresponding operation, and transmits information indicating whether the operation is successfully stopped to the mobile phone by returning the CmdStop message to the mobile phone.

The CmdStop message also includes frame header, load length, check value and payloads, and the payloads include: message type (CmdType, specifically CmdStop), message content, and the like.

### (7) Clock synchronization (CmdSyncClock) message

An initial initiator of the CmdSyncClock message can be the mobile phone, a receiver may be the sensor, and the message is used to send a clock synchronization command to the sensor, instruct the sensor to synchronize an internal clock of the sensor to ensure time consistency, and transmit information, such as sensor number, clock calibration, and whether need to be saved or not, to the sensor. After the sensor confirms that clock synchronization is received, a corresponding clock synchronization operation can be executed.

The CmdSyncClock message also includes frame header, payload length, check value and payload, and the payloads may include: message type (CmdType, specifically CmdSyncClock), sensor number, calibrated clock information, and the like.

### (8) Report status (CmdReportStatus) message

An initial initiator of the CmdReportStatus message may be the sensor, a receiver may be the mobile phone, and the message is used to send a status report from the sensor to the mobile phone, and provide a current status, an operation result or other relevant information, such as a sensor number, a battery level, a signal, and/or impedance of each channel.

The CmdReportStatus message also includes frame header, payload length, check value and payloads, and the payloads may include: a message type (CmdType, specifically CmdReportStatus), sensor number, and part or all of the following information: the battery level, the signal, and/or the impedance of each channel.

The structure of each message body and the content of the contained payloads as described above are merely examples and may contain more or fewer fields. That is, other fields may be added or some fields may be reduced to accommodate different scenarios. Moreover, other message types may exist, and the structures of all these messages may also be defined based on requirements of the actually used transmission protocols.

The mobile phone and the sensor can communicate in a wired or wireless manner, and the mobile phone can acquire various types of information of the sensor through the interaction between the sensor and the mobile phone. After a connection between the mobile phone and the sensor is successfully established, the sensor sends the data packet carrying the human factor data to the mobile phone, and after the receiving end receives the data packet, hexadecimal file storage can be carried out according to the data storage method according to the present disclosure.

In the data storage method according to the present disclosure, the host computer (such as a server, a computer, the mobile phone, an industrial personal computer or other devices) can store the human factor data in the same data format as the data packet of the sensor, and the data format does not need to be changed, so that storage is more efficient, and integrity of the data can be kept. Furthermore, the diversified human factor data with wide purposes is stored in the same storage format, thus facilitating management of the human factor data.

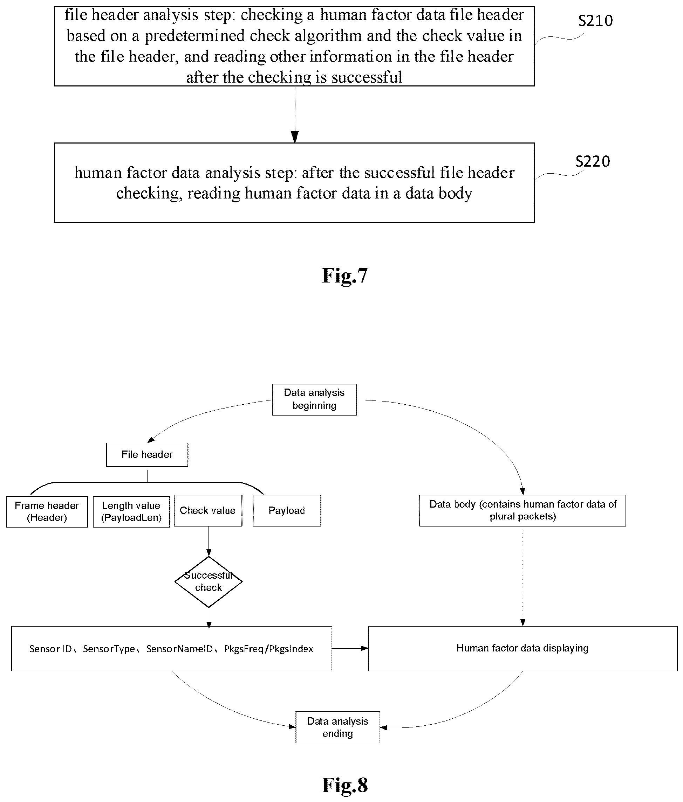

Corresponding to the human factor data file obtained by the foregoing data storage method, the present disclosure further provides a human factor data analysis method for the human factor data file, the analysis method can be carried out in a data processing device serving as the host computer, such as the mobile phone, the computer or the server, and FIG. 7 shows a schematic flow diagram of the human factor data analysis method according to an embodiment of the present disclosure. As shown in FIG. 7, the human factor data file to be analyzed may be selected, and the analysis method including the following steps may be performed on the human factor data file:
step S210: file header analysis step: checking a human factor data file header based on a predetermined check algorithm and a check value in the file header, and reading information in the file header after the checking is successful.

The analyzed information in the file header may include, for example, a frame header and payloads, and the payloads may include, for example: sensor ID (Sensor ID), sensor type (SensorType), sensor name suffix (SensorNameID), and data packet reporting frequency (PkgsFreq).

Step S220: data body analysis step: after the successful file header checking, reading human factor data in a data body.

In this step, corresponding to the foregoing data storage method, if complete data packets containing human factor data are stored in the data body, as shown in FIG. 9, in order to read the human factor data in the data packets, it is necessary to check the data packets one by one, and read the human factor data in all the data packets that are successfully checked, thereby completing analysis of the data body.

If the human factor data extracted from the data packet from the sensor is stored in the data body, as shown in FIG. 8, the human factor data of the data body can be directly read without checking the human factor data.

After the file header and the data body are analyzed, both the payload in the analyzed file header and the human factor data can be visually displayed and can be used for a data query and data analysis.

The data analysis method according to the present disclosure has a high analysis efficiency, high safety, a simple query and a high access efficiency.

Corresponding to the above data storage method, the present disclosure further provides a data storage device (electronic device), including a computer device, the computer device including a processor and a memory, the memory storing computer instructions, the processor being configured to execute the computer instructions stored in the memory, and the data storage device implementing the steps of the data storage method as described above when the computer instructions are executed by the processor.

Corresponding to the above data analysis method, the present disclosure further provides a human factor data analysis device (electronic device), including a computer device, the computer device including a processor and a memory, the memory storing computer instructions, the processor being configured to execute the computer instructions stored in the memory, and the human factor data analysis device implementing the steps of the data analysis method as described above when the computer instructions are executed by the processor.

An embodiment of the present disclosure further provides a computer-readable storage medium having a computer program stored thereon, the computer program being executed by a processor to implement the steps of the foregoing methods. The computer-readable storage medium may be a tangible storage medium, such as a random access memory (RAM), a memory, a read only memory (ROM), an electrically programmable ROM, an electrically erasable programmable ROM, a register, a floppy disk, a hard disk, a removable storage disk, a CD-ROM, or any other form of storage medium known in the art.

An embodiment of the present disclosure further provides a computer program product including computer instructions, the computer instructions, when executed by a processor, implementing the steps of the data storage method and/or the data analysis method described above.

Those of ordinary skill in the art should appreciate that the various exemplary components, systems and methods described in connection with the embodiments disclosed herein may be implemented in hardware, software, or combinations thereof. Whether they are implemented in hardware or software depends upon particular applications and design constraints of the technical solution. Professionals may use different methods for particular applications to achieve the described functions, but such implementations should not be considered beyond the scope of the present disclosure. When implemented in hardware, the elements of the present disclosure may be, for example, an electronic circuit, an application specific integrated circuit (ASIC), suitable firmware, plug-in, a function card, or the like. When implemented in software, the elements of the present disclosure are programs or code segments used to perform required tasks. The programs or the code segments can be stored in a machine-readable medium or transmitted by a data signal carried in a carrier wave over a transmission medium or a communication link.

It is to be understood that the present disclosure is not limited to the particular configurations and processing described above and shown in the drawings. A detailed description of known methods is omitted herein for the sake of brevity. In the above embodiments, several specific steps are described and shown as an example. However, the method processes of the present disclosure are not limited to the specific steps described and shown, and those skilled in the art can make various changes, modifications and additions, or change the order between the steps, after comprehending the spirit of the present disclosure.

Features that are described and/or illustrated with respect to one embodiment may be used in the same way or in a similar way in one or more other embodiments and/or in combination with or instead of the features of the other embodiments in the present disclosure.

The above description is only a preferred embodiment of the present disclosure and is not intended to limit the present disclosure, and various modifications and changes may be made to the embodiment of the present disclosure by those skilled in the art. Any modifications, equivalents and improvements made within the spirit and principle of the present disclosure should be included in the protection scope of the present disclosure.

## Claims

1. A human factor data storage method, comprising:
acquiring a data packet from a sensor, the data packet containing human factor data; and
storing the human factor data in a human factor data file in a predetermined data format, wherein the predetermined data format has a file header and a data body, the file header comprises a frame header, a length value and a check value, the human factor data from the sensor is stored in the data body, and the check value in the file header is obtained based on a predetermined check algorithm.

2. The method according to claim 1, wherein the file header further comprises a file header payload, the file header payload comprises sensor description fields for representing sensor features, and the sensor description fields comprise some or all of the following fields: sensor ID, sensor type, sensor name suffix and data packet reporting frequency;
the length value in the file header is the length of the file header payload.

3. The method according to claim 2, further comprising:
before storing the human factor data from the sensor for the first time, receiving a message from the sensor containing the sensor description fields, and generating the file header of the human factor data file based on the received message.

4. The method according to claim 3, wherein the generating the file header of the human factor data file based on the received message comprises:
taking a message body from the sensor containing the sensor description fields as the file header, the message body comprising the frame header, the length value, the check value and the payload, and the payload comprising the plurality of sensor description fields; or
obtaining the plurality of sensor description fields based on the message from the sensor containing the sensor description fields, filling the frame header of the file header, and filling the length value, the check value and the payload in the file header based on the plurality of sensor description fields to obtain the file header.

5. The method according to any one of claims 1 to 4, wherein the data packet comprises a packet header part and a payload part, the packet header part comprises a frame header, a payload length value and a check value, and the payload part of the data packet comprises the human factor data.

6. The method according to claim 5, wherein the storing the human factor data in a human factor data file in a predetermined data format comprises:
storing the data packet from the sensor as a whole into the data body of the human factor data file, the data body containing one or more data packets.

7. The method according to claim 5, wherein the storing the human factor data in a human factor data file in a predetermined data format comprises:
checking the obtained data packet based on the predetermined data packet check algorithm and the check value in the data packet, and obtaining the human factor data in the data packet after successful checking; and
storing the obtained human factor data in the data body of the human factor data file.

8. The method according to any one of claims 1 to 7, wherein the data packet is a data packet from a human factor data collection sensor containing human factor data;
the human factor data file performs storage based on a hexadecimal format.

9. The method according to claim 1, further comprising:
before acquiring t data packet from the sensor, sending an operation start instruction to the sensor to instruct the sensor to send the data packet containing the human factor data.

10. The method according to claim 3, wherein the message from the sensor containing the sensor description fields is received based on the following interaction process:
sending an initialization message to the sensor and receiving an initialization message from the sensor, the initialization message comprising a frame header region, a payload length region, a check region and a message body part, and the message body part comprising a message type and sensor initialization information comprising the number of connected sensors, type of the sensor and group information; and
based on the received initialization message from the sensor, sending a scan command message to the sensor, the scan command message comprising a frame header region, a payload length region, a check region and a message body part, the message body part comprising a message type and the plurality of sensor description fields, and the plurality of sensor description fields comprising some or all of the following fields: sensor ID, sensor type, sensor name suffix and data packet reporting frequency.

11. A human factor data analysis method, the human factor data being stored in a human factor data file in a predetermined format, the predetermined data format having a file header and a data body, the file header comprising a frame header, a length value and a check value, the data body comprising the human factor data, and the method comprising:
a file header analysis step: checking the human factor data file header based on a predetermined check algorithm and the check value in the file header, and reading other information in the file header after the checking is successful; and
a data body analysis step: after the successful file header checking, reading the human factor data in the data body.

12. The method according to claim 11, wherein the data body contains at least one data packet containing the human factor data, the data packet containing the human factor data comprises a packet header and a payload, the packet header comprises a frame header, a payload length value and a check value, a payload of the data packet comprises the human factor data, and the human factor data analysis step comprises:
checking the data packet in the data body based on a predetermined data packet check algorithm and the check value in the data packet, and reading the human factor data in the data packet after successful checking.

13. The method according to claim 11, wherein the file header further comprises a file header payload, and the file header payload comprises sensor description fields; and
the method further comprises: displaying the read information in the file header and the human factor data in the data body on a display apparatus.

14. An electronic device, comprising a processor, a memory and computer instructions stored on the memory, the processor being configured to execute the computer instructions, and the device implementing the steps of the method according to any one of claims 1 to 13 when the computer instructions are executed.

15. A computer-readable storage medium having computer instructions stored therein, the computer instructions, when executed by a processor, implementing the steps of the method according to any one of claims 1 to 13.
